# EUROPÄISCHE PATENTSCHRIFT

(11) **EP 3 534 868 B1**
(45) Veröffentlichungstag und Bekanntmachung des Hinweises auf die Patenterteilung: **20.10.2021**
(21) Anmeldenummer: 17804059.8
(22) Anmeldetag: 03.11.2017
(51) Int. Cl.: A61K 8/02, A45D 29/00, D04H 1/40, A61Q 3/04

(54) **KOSMETISCHES PAD UND VERFAHREN ZUR HERSTELLUNG EINES KOSMETISCHEN PADS**
COSMETIC PAD AND METHOD FOR PRODUCING A COSMETIC PAD
TAMPON COSMÉTIQUE ET PROCÉDÉ DE FABRICATION D'UN TAMPON COSMÉTIQUE

(30) Priorität: 03.11.2016 DE 102016013067
(43) Veröffentlichungstag der Anmeldung: 11.09.2019
(73) Patentinhaber: Man, Kai-Wing, 68159 Mannheim (DE)
(72) Erfinder: Man, Kai-Wing, 68159 Mannheim (DE)
(74) Vertreter: Sattler de Sousa e Brito, Clara
(86) Internationale Anmeldenummer: PCT/EP2017/001276
(87) Internationale Veröffentlichungsnummer: WO 2018/082812

(56) Entgegenhaltungen:
- WO-A1-2016/020236
- US-A1- 2003 127 104
- US-A1- 2005 238 698
- US-B1- 6 492 307

## Beschreibung

Die Erfindung betrifft ein kosmetisches Pad und ein Verfahren zur Herstellung eines kosmetischen Pads.

### HINTERGRUND DER ERFINDUNG

Für kosmetische Anwendungen, zum Beispiel zum Entfernen von Schminke (Make-up), werden oft Wattepads verwendet. Wattepads bestehen üblicherweise aus Fasern, die in einem losen Gefüge auf Grund der Haftung der Fasern untereinander zusammengehalten werden. Bekannt ist Baumwollfasern für Wattepads zu verwenden.

### ZUSAMMENFASSUNG DER ERFINDUNG

Es ist Aufgabe der vorliegenden Erfindung, ein verbessertes Wattepad anzugeben, so dass das Entfernen von kosmetischen Überresten auf Haut oder Nägeln vereinfacht wird.

Die erfindungsgemäße Lösung der Aufgabe erfolgt durch die Merkmale der unabhängigen Ansprüche.

Erfindungsgemäß wird ein kosmetisches Pad zur Entfernung von Nagellackentferner oder Make-up-Entferner angegeben, wobei das kosmetische Pad einen Saugkern (2) und eine Vliesschicht (1) auf beiden Seiten einer flüssigkeitsundurchlässigen Schicht (6) aufweist. Die Vliesschicht dient der Aufnahme und Speicherung von Flüssigkeit. Es nimmt Flüssigkeiten oder flüssige kosmetische Überreste beispielsweise auf Haut oder Fingernägeln auf und speichert diese. Dies gilt auch für lösbare kosmetische Überreste, die am Vlies haften bleiben können. Die Vliesschicht hat die Funktion einer Absorberschicht des kosmetischen Pads. Es kann eine Absorberschicht sein. Der Saugkern speichert Flüssigkeiten oder flüssige kosmetische Überreste. Ebenso dient der Saugkern einer angemessenen Abgabe der Flüssigkeit oder flüssiger kosmetischer Überreste. In manchen Ausführungsbeispielen weist der Saugkern Zellstoff-Flocken und ein Superabsorbergranulat auf. Ein Superabsorbergranulat, beispielsweise Superabsorbent Polymers (SAP), ist in der Lage ein Vielfaches an Flüssigkeit aufzusaugen, beispielsweise Wasser oder wässrige Lösungen.

Das erfindungsgemäße kosmetische Pad ist in einer Weiterentwicklung perforiert, sodass es in zwei Teile entteilt werden kann. Die Vliesschicht kann dazu eine Prägung wie beispielsweise ein Blumenmuster oder Noppen aufweisen, die in das Vliesmaterial geprägt sind. In manchen der folgenden Weiterentwicklungen und Ausführungsbeispielen bewirkt diese Prägung zusätzlich eine Adhäsion der Zellstoffflocken an eine flüssigkeitsundurchlässige Schicht, sodass die Zellstoff-Flocken sich nicht lose zwischen der Vliesschicht und der flüssigkeitsundurchlässigen Schicht verteilen können. Des Weiteren kann sich die Speicherung von Flüssigkeiten verbessern in den Bereichen in denen die Vliesschicht geprägt wurde, da die Fasern der Schichten in diesem Bereich durch die Prägung enger aneinander liegen und die Speicherung von Flüssigkeiten begünstigen. Die Vliesschicht(en) können voneinander abweichende Oberflächenstrukturen haben. Beispielsweise verbessert eine grobere Struktur das Abreiben und die Beseitigung von groberem Schmutz auf Haut oder Nägeln, wobei eine feinere Struktur eher zum Nachpolieren geeignet ist.

Das kosmetische Pad sieht in einer Weiterentwicklung vor, dass das Pad zwischen Saugkern und Vliesschicht eine Verteilerauflage aufweist. Die Verteilerauflage dient der schnellen Weiterleitung der Flüssigkeit oder flüssiger kosmetischer Überreste an den Saugkern. Der Saugkern speichert die Flüssigkeit oder flüssiger kosmetischer Überreste. Ebenso dient er einer angemessenen Abgabe der Flüssigkeit oder flüssiger kosmetischer Überreste zurück in die Vliesschicht.

Gemäß einer anderen Weiterentwicklung des kosmetischen Pads ist vorgesehen, dass das Pad zwischen der Vliesschicht und der Verteilerauflage eine Verklebung aufweist. Die Verklebung ermöglicht eine verbesserte Verbindung zwischen der Vliesschicht und der Verteilerauflage. Die Verklebung erfolgt beispielsweise über einen oder eine Kombination von Klebstoffen.

In einer Weiterentwicklung des kosmetischen Pads ist vorgesehen, dass das Pad eine flüssigkeitsundurchlässige Schicht, insbesondere eine Folie oder ein Papier, einen Saugkern und eine Vliesschicht aufweist. Die flüssigkeitsundurchlässige Schicht kann als Schutzfolie ausgebildet sein. Die flüssigkeitsundurchlässige Schicht begünstigt die Speicherung und Abgabe von Flüssigkeit im Saugkern. Die flüssigkeitsundurchlässige Schicht, insbesondere die Folie oder das Papier, ermöglicht eine voneinander getrennte doppelseitige Verwendung des Pads.

Gemäß einer anderen Weiterentwicklung des kosmetischen Pads ist vorgesehen, dass auch auf der freiliegenden Seite der flüssigkeitsundurchlässigen Schicht ein weiterer Saugkern und eine weitere Vliesschicht angeordnet sind. Um ein noch besseres Entfernungsergebnis zu erzielen, trennt in manchen Ausführungsbeispielen die flüssigkeitsundurchlässige Schicht zwei Saugkernschichten des Saugkerns voneinander, um eine optimale beidseitige Benutzung zu gewährleisten. Mit anderen Worten, die flüssigkeitsundurchlässige Schicht ist in einem inneren, mittleren Bereich des Saugkerns angeordnet.

In einer Weiterentwicklung des kosmetischen Pads ist vorgesehen, dass der Saugkern und die unterschiedlichen Schichten im Randbereich verbunden sind. Die Vliesschicht und der Saugkern werden in einem Randbereich unter Druck zusammengepresst. Durch das Zusammenpressen entsteht eine dünne Schicht. Der Randbereich oder Rand bildet eine geschlossene Fläche. Durch den Rand ist eine Haftung der unterschiedlichen Schichten gewährleistet, um die besondere Saugfähigkeit im Inneren des Pads aufrecht zu erhalten und um eine Trennung der Schichten durch das Reiben auf einer Fläche zu vermeiden. Auch dient der Rand zusätzlich dazu, ein fusselfreies Ergebnis zu erzielen. Das Zusammenpressen erfolgt als eine Mischung von verkleben und verprägen, wenn die Vliesschicht Kunststoffanteile aufweist, so dass die Vliesschicht(en) und der Saugkern im Randbereich durch ein heißes Aufeinanderpressen miteinander zumindest teilweise verschmelzen.

Gemäß einer anderen Weiterentwicklung des kosmetischen Pads ist vorgesehen, dass das Pad mit einer Flüssigkeit insbesondere Nagellackentferner oder Make-up-Entferner getränkt ist. Durch die ausgezeichnete Aufnahme und Speicherung von Flüssigkeit kann das kosmetische Pad insbesondere den Nagellackentferner oder Make-up-Entferner hervorragend speichern. Insbesondere der Saugkern ermöglicht nicht nur eine Speicherung, sondern auch gleichzeitig eine Abgabe der Flüssigkeit durch die Vliesschicht an Haut, Fingernägel oder Zehennägel. Die Abgabe der Flüssigkeit kann beispielsweise durch Druck auf das Pad erfolgen.

Gemäß einer Weiterentwicklung des kosmetischen Pads ist vorgesehen, dass das Pad eine runde, ovale oder rechteckige Form aufweist. Eine solche Form des Pads begünstigt die Handhabung und das Auftragen von Flüssigkeiten bzw. das Aufnehmen von Flüssigkeiten oder kosmetischen Überresten. Bei einer rechteckigen Form weist das Pad eine Länge und eine Breite von ca. 5,5 oder ca. 4,5 cm auf, bei einer Toleranz von jeweils 0,2-0,5 cm. Bei einer ovalen Form des Pads beträgt die Länge ca. 9,0 cm und die Breite ca. 7,2 cm, bei einer Toleranz von jeweils 0,2-0,5 cm.

In einer Weiterentwicklung des kosmetischen Pads ist vorgesehen, dass das Pad einen Durchmesser, eine Länge oder eine Breite von 2-10 cm insbesondere von 3-6 cm insbesondere von 5,5 oder 4,5 cm aufweist. Die Maßangaben enthalten eine Toleranz von jeweils 0,2-0,5 cm.

In einer anderen Weiterentwicklung des kosmetischen Pads ist vorgesehen, dass das Pad eine Dicke von 3,0 mm bis 6,0 mm, insbesondere von 3,5 mm bis 5,0 mm oder von 3,0 mm bis 4,5 mm aufweist. Die Maßangaben enthalten eine Toleranz von jeweils etwa 0,5-1,0 mm.

Erfindungsgemäß wird auch ein Verfahren zur Herstellung insbesondere eines genannten kosmetischen Pads angegeben.

Vorteilhafte Weiterbildungen der Erfindung ergeben sich aus den Unteransprüchen.

### KURZE BESCHREIBUNG DER FIGUREN

Weitere Einzelheiten der Erfindung sind den Ausführungsbeispielen zu entnehmen, die im Folgenden anhand der Figuren beschrieben wird. Es zeigen:
- Figur 1:: ein Referenzbeispiel eines kosmetischen Pads,
- Figur 2:: das kosmetische Pad aus Fig. 1 in einer perspektivischen Ansicht,
- Figur 3:: ein weiteres Referenzbeispiel eines kosmetischen Pads,
- Figur 4:: das kosmetische Pad aus Fig. 3 in einer perspektivischen Ansicht,
- Figur 5:: ein weiteres Ausführungsbeispiel eines kosmetischen Pads,
- Figur 6:: das kosmetische Pad aus Fig. 5 in einer perspektivischen Ansicht,
- Figur 7:: ein weiteres Ausführungsbeispiel eines kosmetischen Pads,
- Figur 8:: ein weiteres Ausführungsbeispiel eines kosmetischen Pads,
- Figur 9:: zwei Ausführungsbeispiele eines Randbereichs eines kosmetischen Pads.

### AUSFÜHRLICHE BESCHREIBUNG DER FIGUREN

Im Folgenden werden exemplarische Ausführungsformen der Erfindung unter Bezugnahme auf die beiliegenden Zeichnungen beschrieben:

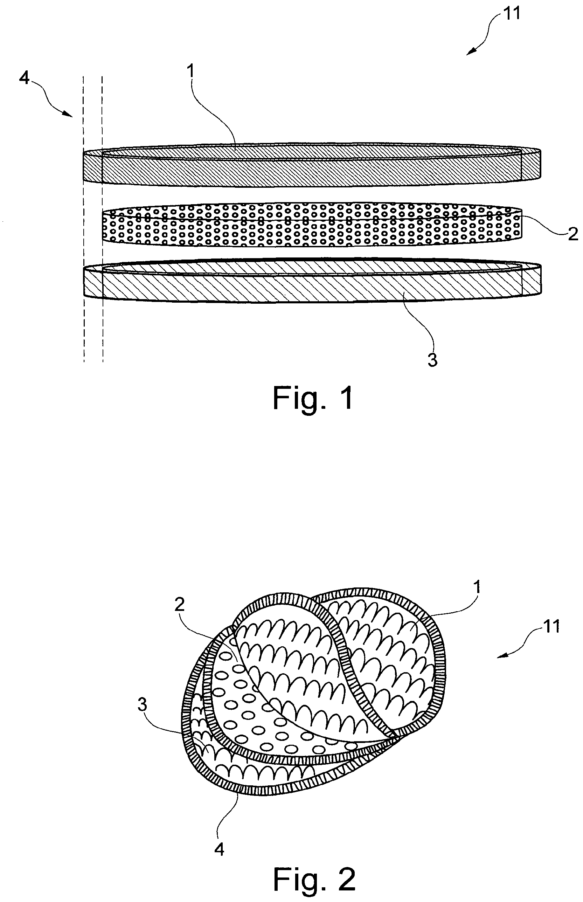

Figur 1 zeigt ein Ausführungsbeispiel eines kosmetischen Pads 11. Die einzelnen Schichten des kosmetischen Pads 11 sind zur besseren Übersicht voneinander beabstandet gezeigt.

Das Obermaterial des kosmetischen Pads 11 weist eine Vliesschicht 1 auf, welche die Funktion der Aufnahme und Speicherung von Flüssigkeit hat. Die Vliesschicht 1 grenzt an einen Saugkern 2, welcher die Flüssigkeit speichert. Ebenso dient der Saugkern einer angemessenen Abgabe der Flüssigkeit zurück in die Vliesschicht 1 und in die Vliesschicht 3. In Figur 1 ist ein kosmetisches Pad 11 mit einer oberen Vliesschicht 1 und einer unteren Vliesschicht 3 gezeigt. Es gibt jedoch auch Ausführungsbeispiele in denen das kosmetische Pad 11 nur eine Vliesschicht 1, 3 und einen Saugkern 2 aufweist.

Die Vliesschichten 1, 3 werden im Randbereich 4 unter Druck fest zusammengepresst, sodass der Randbereich 4 als eine dünne Schicht ausgebildet ist und eine geschlossene Fläche bildet. Durch den Rand 4 wird eine Haftung der verschiedenen Schichten 1, 2, 3 gewährleistet, um die besondere Saugfähigkeit im Inneren des Pads aufrecht zu erhalten und um eine Trennung der Schichten 1, 2, 3 durch beispielsweise das Reiben auf einer Fläche zu vermeiden. Auch dient der Rand 4 zusätzlich dazu, ein absolut fusselfreies Ergebnis zu erzielen.

In einem Ausführungsbeispiel, in dem die eine oder mehrere Vliesschichten 1, 3 Kunststoffanteile aufweist, erfolgt das Zusammenpressen als eine Mischung von Verkleben und Verprägen, so dass die Vliesschicht(en) 1, 3 und der Saugkern 2 im Randbereich 4 durch ein heißes Aufeinanderpressen miteinander zumindest teilweise verschmelzen.

Das kosmetische Pad hat einen Durchmesser von ca. 5,5 cm und eine Dicke von ca. 3,0 mm bis 4,5 mm.

Figur 2 zeigt eine perspektivische Ansicht des kosmetischen Pads 11 aus Figur 1. Die einzelnen Vliesschichten 1, 3 als auch der Saugkern 2 sind zum besseren Verständnis einseitig aufgefächert gezeigt. Gut sichtbar ist auch der Randbereich 4 der einzelnen Schichten des kosmetischen Pads 11.

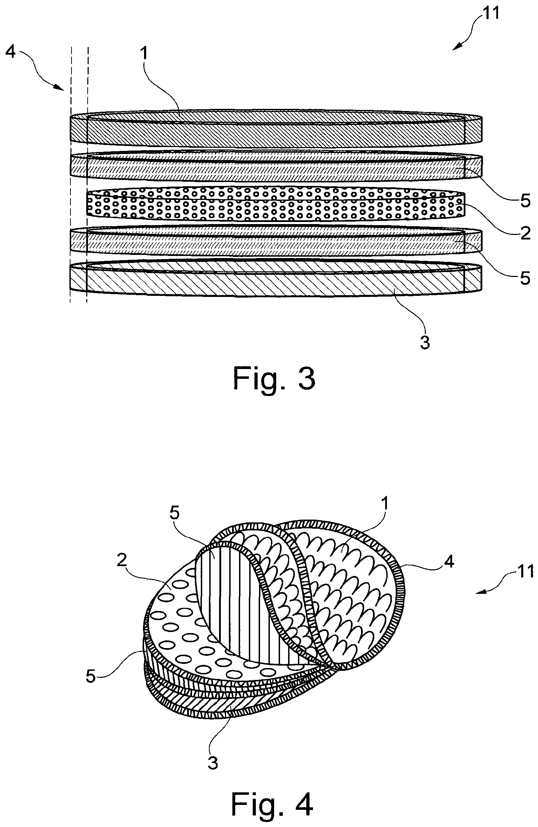

Figur 3 zeigt ein weiteres Ausführungsbeispiel eines kosmetischen Pads 11. Die einzelnen Schichten des kosmetischen Pads 11 sind zur besseren Übersicht voneinander beabstandet gezeigt.

Das Obermaterial des kosmetischen Pads 11 weist eine Vliesschicht 1 auf, und hat die Funktion der Aufnahme und Speicherung von Flüssigkeit. Die Vliesschicht 1 grenzt an eine Verteilerauflage 5, welche für die schnelle Weiterleitung der Flüssigkeit an den Saugkern 2 sorgt. Der Saugkern 2 speichert die Flüssigkeit, ebenso dient er einer angemessenen Abgabe der Flüssigkeit zurück in die obere/untere Verteilerauflage 5 und obere/untere Vliesschicht 1, 3. Es gibt auch Ausführungsbeispiele in denen das kosmetische Pad 11 nur eine Vliesschicht 1, 3, eine Verteilerauflage 5 und einen Saugkern 2 aufweist.

Die Vliesschichten 1, 3, und die Verteilerauflage 5 (oder Verteilerschicht) werden im Randbereich 4 unter Druck fest zusammengepresst, sodass eine dünne Schicht entsteht und der Rand 4 eine geschlossene Fläche bildet. Durch den Rand 4 wird eine Haftung der verschiedenen Schichten 1, 2, 3, 5 gewährleistet, um die besondere Saugfähigkeit im Inneren des Pads aufrecht zu erhalten und um eine Trennung der Schichten 1, 2, 3, 5 durch beispielsweise das Reiben auf einer Fläche zu vermeiden. Auch dient der Rand 4 zusätzlich dazu, ein absolut fusselfreies Ergebnis zu erzielen.

Das kosmetische Pad hat einen Durchmesser von ca. 5,5 cm und eine Dicke von ca. 4,0 mm bis 5,0 mm.

In einem Ausführungsbeispiel, in dem die eine oder mehrere Vliesschichten 1, 3 Kunststoffanteile aufweist, erfolgt das Zusammenpressen als eine Mischung von Verkleben und Verprägen, so dass die Vliesschicht(en) 1, 3 und die Verteilerauflage 5 und der Saugkern 2 im Randbereich 4 durch ein heißes Aufeinanderpressen miteinander zumindest teilweise verschmelzen.

Figur 4 zeigt eine perspektivische Ansicht des kosmetischen Pads 11 aus Figur 3. Die einzelnen Vliesschichten 1, 3, die Verteilerauflage 5 als auch der Saugkern 2 sind zum besseren Verständnis einseitig aufgefächert gezeigt. Gut sichtbar ist auch der Randbereich 4 der einzelnen Schichten des kosmetischen Pads 11.

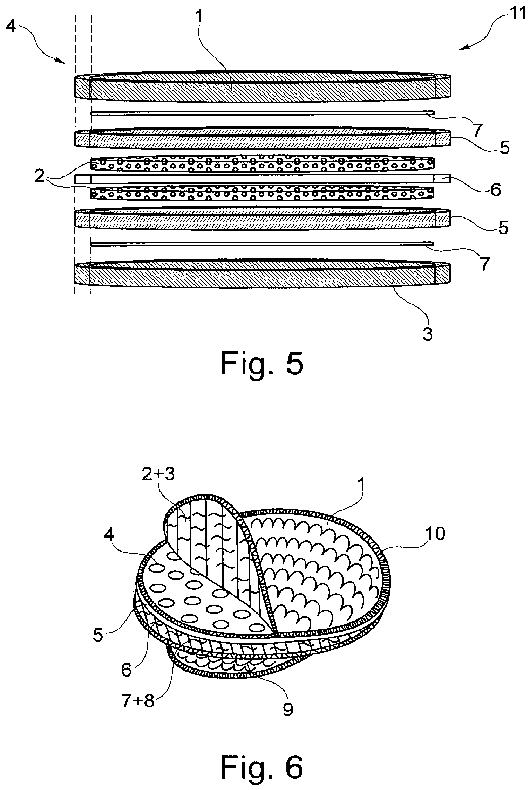

Figure 5 zeigt ein weiteres Ausführungsbeispiel eines kosmetischen Pads 11. Die einzelnen Schichten des kosmetischen Pads 11 sind zur besseren Übersicht voneinander beabstandet gezeigt.

Das Obermaterial des kosmetischen Pads 11 weist eine Vliesschicht 1 auf, und hat die Funktion der Aufnahme und Speicherung von Flüssigkeit. Die Vliesschicht 1, auch Absorberschicht genannt, grenzt an eine Verklebung 7. Darauf folgt eine Verteilerauflage 5, die der schnellen Weiterleitung der Flüssigkeit an den Saugkern 2 dient. Der Saugkern 2 speichert die Flüssigkeit, ebenso dient er einer angemessenen Abgabe der Flüssigkeit zurück in die obere/untere Verteilerauflage 5 und obere/untere Vliesschicht 1, 3. Der Saugkern 2 weist einen Zellstoff und ein Supergranulat auf. Es gibt auch Ausführungsbeispiele in denen das kosmetische Pad 11 nur eine Vliesschicht 1, 3, eine Verklebung 7, eine Verteilerauflage 5 und einen Saugkern 2 aufweist.

Um ein besseres Entfernungsergebnis zu erzielen, trennt eine flüssigkeitsundurchlässige Schicht 6 zwei Schichten des Saugkerns 2 voneinander, um eine optimale beidseitige Benutzung gewährleisten zu können. Die flüssigkeitsundurchlässige Schicht 6 kann mittig zwischen den zwei Schichten des Saugkerns 2 angeordnet sein. Die flüssigkeitsundurchlässige Schicht 6 ist in diesem Ausführungsbeispiel eine Folie, kann jedoch auch ein Papier sein.

Die Vliesschichten 1, 3, die Verteilerauflage 5 und die flüssigkeitsundurchlässige Schicht 6 werden im Randbereich 4 unter Druck fest zusammengepresst, sodass eine dünne Schicht entsteht und der Rand 4 eine geschlossene Fläche bildet. Durch den Rand 4 wird eine Haftung der verschiedenen Schichten 1, 2, 3, 5, 6 gewährleistet, um die besondere Saugfähigkeit im Inneren des Pads aufrecht zu erhalten und um eine Trennung der Schichten 1, 2, 3, 5, 6 durch beispielsweise das Reiben auf einer Fläche zu vermeiden. Auch dient der Rand 4 zusätzlich dazu, ein absolut fusselfreies Ergebnis zu erzielen.

Das kosmetische Pad hat einen Durchmesser von ca. 5,5 cm und eine Dicke von ca. 5,0 bis 6,0 mm.

In einem Ausführungsbeispiel, in dem die eine oder mehrere Vliesschichten 1, 3 Kunststoffanteile aufweist, erfolgt das Zusammenpressen als eine Mischung von Verkleben und Verprägen, so dass die Vliesschicht(en) 1, 3, die Verteilerauflage 5, die flüssigkeitsundurchlässige Schicht 6 und der Saugkern 2 im Randbereich 4 durch ein heißes Aufeinanderpressen miteinander zumindest teilweise verschmelzen. Die flüssigkeitsundurchlässige Schicht 6, welche als Folie ausgebildet sein kann, weist in manchen Ausführungsbeispielen Kunststoffanteile auf, sodass durch das heiße Aufeinanderpressen der Schichten, deren Verklebung, Prägung verbessert werden kann.

Figur 6 zeigt eine perspektivische Ansicht des kosmetischen Pads 11 aus Figur 5. Die einzelnen Vliesschichten 1, 3, die Verteilerauflage 5 mit der Verklebung 7, die flüssigkeitsundurchlässige Schicht 6, als auch der zweiteilige Saugkern 2 sind zum besseren Verständnis einseitig aufgefächert gezeigt. Gut sichtbar ist auch der Randbereich 4 der einzelnen Schichten des kosmetischen Pads 11.

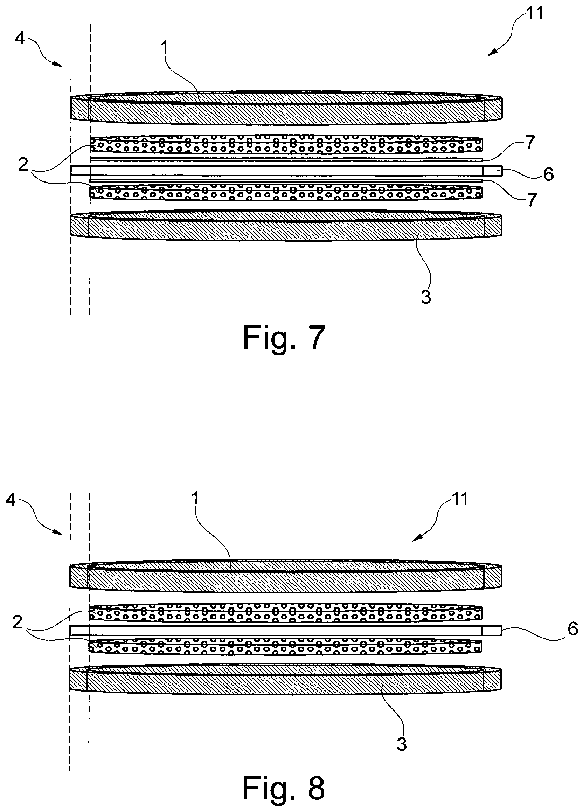

Figur 7 zeigt ein weiteres Ausführungsbeispiel eines kosmetischen Pads 11. Die einzelnen Schichten des kosmetischen Pads 11 sind zur besseren Übersicht voneinander beabstandet gezeigt.

Das Obermaterial des kosmetischen Pads 11 weist eine Vliesschicht 1 auf und hat die Funktion der Aufnahme und Speicherung von Flüssigkeit. Die Vliesschicht 1, auch Absorberschicht genannt, grenzt an eine Schicht eines Saugkerns 2. Darauf folgt eine Verklebung 7 und eine flüssigkeitsundurchlässige Schicht 6, eine weitere Verklebung 7, eine weitere Schicht des Saugkerns 2 und eine Vliesschicht 3.

Um ein besseres Entfernungsergebnis zu erzielen, trennt eine flüssigkeitsundurchlässige Schicht 6 die zwei Schichten des Saugkerns 2 voneinander, um eine optimale beidseitige Benutzung gewährleisten zu können. Die flüssigkeitsundurchlässige Schicht 6 ist in diesem Ausführungsbeispiel eine Folie, kann jedoch auch ein Papier sein. Der Saugkern 2 speichert die Flüssigkeit und dient einer angemessenen Abgabe der Flüssigkeit zurück in die obere/untere Vliesschicht 1, 3.

In diesem Ausführungsbeispiel weist der Saugkern 2 nur Zellstoff-Flocken auf. Im Saugkern ist kein Superabsorber vorhanden.

Wie auch bei Figur 5 beschrieben, werden die Vliesschicht 1, 3 und die Folie 6 im Randbereich 4 unter Druck fest zusammengepresst, sodass eine dünne Schicht entsteht und der Rand 4 eine geschlossene Fläche bildet. Durch den Rand 4 wird eine Haftung der verschiedenen Schichten gewährleistet, um die besondere Saugfähigkeit im Inneren des Pads 11 aufrecht zu erhalten und um eine Trennung der Schichten durch das Reiben auf einer Fläche zu vermeiden. Auch dient der Rand 4 zusätzlich dazu, ein absolut fusselfreies Ergebnis zu erzielen.

Das kosmetische Pad hat einen Durchmesser von ca. 5,5 cm und eine Dicke von ca. 3,5 bis 5,0 mm.

Figur 8 zeigt ein weiteres Ausführungsbeispiel eines kosmetischen Pads 11. Die einzelnen Schichten des kosmetischen Pads 11 sind zur besseren Übersicht voneinander beabstandet gezeigt.

Der Aufbau des kosmetischen Pads 11 ist ähnlich dem Ausführungsbeispiel aus Figur 7 und 6, jedoch ohne Verklebung, so dass sich eine Abfolge von Vliesschicht 1, erster Teil des zweiteiligen Saugkerns 2, flüssigkeitsundurchlässige Schicht 6, zweiter Teil des zweiteiligen Saugkerns 2 und Vliesschicht 3 ergibt. Die Beschreibung von Figur 7 gilt entsprechend für Figur 8.

Wie im Ausführungsbeispiel aus Figur 7 weist der Saugkern 2 nur Zellstoff-Flocken auf. Durch Adhäsion der Zellstoff-Flocken auf der flüssigkeitsundurchlässige Schicht 6 kann eine Verklebung entfallen.

Das kosmetische Pad hat einen Durchmesser von ca. 5,5 cm und eine Dicke von ca. 3,5 bis 5,5 mm.

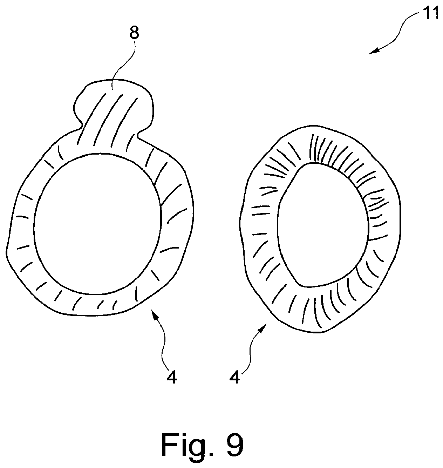

Figur 9 zeigt zwei Ausführungsbeispiele eines Randbereichs 4 eines kosmetischen Pads. Der Randbereich 4 ist wie in den vorherigen Figuren beschrieben und zusätzlich breit und/oder geriffelt gepresst. Dies ermöglicht einen besonders guten Zugang in runde Ecken oder für den Rand der Fingernägel oder Zehennägel.

Eine dieser Ausführungsbeispiele mit breitem geprägten Rand 4 weist eine Lasche 8 um besonders einfach Nagellack am Nagelrand zu entfernen.

Es ist zu beachten, dass die in diesem Dokument beschriebenen Verfahren, Vorrichtungen und Systeme sowohl alleine, als auch in Kombination mit anderen in diesem Dokument beschriebenen Verfahren, Vorrichtungen und Systemen verwendet werden können. Des Weiteren können jegliche Aspekte der in diesem Dokument beschriebenen Verfahren, Vorrichtung und Systemen in vielfältiger Weise miteinander kombiniert werden. Insbesondere können die Merkmale der Ansprüche in vielfältiger Weise miteinander kombiniert werden.

Die Erfindung ist anhand der Zeichnungen und der obigen Beschreibung ausführlich beschrieben. Diese Erfindung kann jedoch in vielen verschiedenen Formen ausgeführt werden und sollte nicht als auf die hier dargelegten Ausführungsformen begrenzt ausgelegt werden; vielmehr sind diese Ausführungsformen vorgesehen, damit diese Offenbarung gründlich und vollständig ist, und decken den Schutzumfang der Erfindung für einen Fachmann vollständig ab. Die Terminologie, die in der ausführlichen Beschreibung der in den beiliegenden Zeichnungen dargestellten Ausführungsformen verwendet wird, soll für die Erfindung nicht einschränkend sein. In den Zeichnungen beziehen sich gleiche Zeichen auf gleiche Elemente.

## Patentansprüche

1. Kosmetisches Pad zur Entfernung von Nagellackentferner oder Make-up-Entferner, **dadurch gekennzeichnet, dass** es eine flüssigkeitsundurchlässige Schicht (6), einen Saugkern (2) und eine Vliesschicht (1) aufweist, und dass auch auf der freiliegenden Seite der flüssigkeitsundurchlässigen Schicht ein weiterer Saugkern (2) und eine weitere Vliesschicht (3) angeordnet sind.

2. Kosmetisches Pad nach Anspruch 1, **dadurch gekennzeichnet, dass** es zwischen Saugkern und Vliesschicht eine Verteilerauflage (5) aufweist.

3. Kosmetisches Pad nach Anspruch 1 oder 2, **dadurch gekennzeichnet, dass** die flüssigkeitsundurchlässige Schicht (6) eine Folie oder ein Papier ist.

4. Kosmetisches Pad nach einem der vorhergehenden Ansprüche, **dadurch gekennzeichnet, dass** es zwischen der Vliesschicht und der Verteilerauflage oder zwischen dem Saugkern und der flüssigkeitsundurchlässigen Schicht eine Verklebung (7) aufweist.

5. Kosmetisches Pad nach einem der vorhergehenden Ansprüche, **dadurch gekennzeichnet, dass** der Saugkern und die unterschiedlichen Schichten im Randbereich (4) verbunden sind.

6. Kosmetisches Pad nach einem der vorhergehenden Ansprüche, **dadurch gekennzeichnet, dass** das Pad mit einer Flüssigkeit insbesondere Nagellackentferner oder Make-up-Entferner getränkt ist.

7. Kosmetisches Pad nach einem der vorhergehenden Ansprüche, **dadurch gekennzeichnet, dass** das Pad eine runde, ovale oder rechteckige Form aufweist.

8. Kosmetisches Pad nach einem der vorhergehenden Ansprüche, **dadurch gekennzeichnet, dass** das Pad einen Durchmesser, eine Länge oder eine Breite von 2-10 cm insbesondere von 3-6 cm insbesondere von 5,5 cm oder 4,5 cm aufweist.

9. Kosmetisches Pad nach einem der vorhergehenden Ansprüche, **dadurch gekennzeichnet, dass** das Pad eine Dicke von 3,0 mm bis 6,0 mm, insbesondere von 3,5 mm bis 5,0 mm oder von 3,0 mm bis 4,5 mm aufweist.

10. Verfahren zur Herstellung eines kosmetischen Pads insbesondere nach einem der Ansprüche 1 bis 9.

## Claims

1. Cosmetic pad for removing nail polish remover or make-up remover, **characterised in that** it has a fluid-impenetrable layer (6), an absorbent core (2) and a non-woven layer (1), and that a further absorbent core (2) and a further non-woven layer (3) are also arranged on the exposed side of the fluid-impenetrable layer.

2. Cosmetic pad according to claim 1, **characterised in that** it has a distribution layer (5) between absorbent core and non-woven layer.

3. Cosmetic pad according to claim 1 or 2, **characterised in that** the fluid-impenetrable layer (6) is a film or a paper.

4. Cosmetic pad according to any one of the preceding claims, **characterised in that** there is an adhesive (7) between the non-woven layer and the distribution layer or between the absorbent core and the fluid-impenetrable layer.

5. Cosmetic pad according to any one of the preceding claims, **characterised in that** the absorbent core and the different layers are joined together around the edge area (4).

6. Cosmetic pad according to any one of the preceding claims, **characterised in that** the pad is soaked with a fluid, in particular a nail polish remover or a make-up remover.

7. Cosmetic pad according to any one of the preceding claims, **characterised in that** the pad has a round, oval or rectangular shape.

8. Cosmetic pad according to any one of the preceding claims, **characterised in that** the pad has a dimension wherein its length or width can be from 2-10 cm, in particular 3-6 cm and more particularly 5.5 cm or 4.5 cm.

9. Cosmetic pad according to any one of the preceding claims, **characterised in that** the thickness of the pad is from 3.0 mm to 6.0 mm, in particular from 3.5 mm to 5.0 mm or from 3.0 mm to 4.5 mm.

10. Method of producing a cosmetic pad particularly according to any one of claims 1 to 9.

## Revendications

1. Pad cosmétique pour enlever du dissolvant de vernis à ongle ou du démaquillant, **caractérisé en ce qu'**il présente une couche imperméable au liquide (6), un cœur absorbant (2) et une couche de non-tissé (1), et **en ce qu'**un autre cœur absorbant (2) et une autre couche de non-tissé (3) sont disposés sur le côté exposé de la couche imperméable au liquide.

2. Pad cosmétique selon la revendication 1, **caractérisé en ce qu'**il présente un revêtement de distribution (5) entre le cœur absorbant et la couche de non-tissé.

3. Pad cosmétique selon la revendication 1 ou 2, **caractérisé en ce que** la couche imperméable au liquide (6) est un film ou un papier.

4. Pad cosmétique selon l'une des revendications précédentes, **caractérisé en ce qu'**il présente un collage (7) entre la couche de non-tissé et le revêtement de distribution ou entre le cœur absorbant et la couche imperméable au liquide.

5. Pad cosmétique selon l'une des revendications précédentes, **caractérisé en ce que** le cœur absorbant et les différentes couches sont reliés au niveau du rebord (4).

6. Pad cosmétique selon l'une des revendications précédentes, **caractérisé en ce que** le pad est imbibé d'un liquide, notamment de dissolvant de vernis à ongle ou de démaquillant.

7. Pad cosmétique selon l'une des revendications précédentes, **caractérisé en ce que** le pad est de forme ronde, ovale ou rectangulaire.

8. Pad cosmétique selon l'une des revendications précédentes, **caractérisé en ce que** le pad présente un diamètre, une longueur ou une largeur de 2-10 cm, notamment de 3-6 cm, notamment de 5,5 cm ou de 4,5 cm.

9. Pad cosmétique selon l'une des revendications précédentes, **caractérisé en ce que** le pad présente une épaisseur de 3,0 mm à 6,0 mm, notamment de 3,5 mm à 5,0 mm ou de 3,0 mm à 4,5 mm.

10. Procédé de fabrication d'un pad cosmétique, notamment selon l'une des revendications 1 à 9.
